# EUROPEAN PATENT APPLICATION

(11) **EP 4 101 934 A1**
(43) Date of publication of application: **14.12.2022**
(21) Application number: 21305779.7
(22) Date of filing: 08.06.2021
(51) Int. Cl.: C12Q 1/6844, C12Q 1/70

(54) **METHOD AND KITS FOR DETECTING SARS VIRAL INFECTION IN A BIOLOGICAL SAMPLE**

(71) Applicant: Centre de Coopération Internationale en Recherche Agronomique pour le Développement (CIRAD), 75116 Paris (FR); Université de La Réunion, 97715 Saint-Denis Cedex 9 (FR)
(72) Inventor: ROBENE, Isabelle, 97427 L Etang-Salé (FR); JOUEN, Emmanuel, 97410 Saint-Pierre (FR); HASCOAT, Jérémy, 97480 Saint-Joseph (FR); CETRE-SOSSAH, Catherine, 34920 LE CRES (FR); REYNAUD, Bernard, 97410 Saint-Pierre (FR); LEBON, Véronique, 97418 Le Tampon (FR); FENELON, Babbitha, 97410 Saint-Pierre (FR)
(74) Representative: LLR

(57) **Abstract**

The invention relates to a method of detecting the presence of a SARS coronavirus nucleic acid, comprising:
- contacting the biological sample with a sample buffer comprising a set of loop-mediated isothermal amplification primers specific for the SARS nucleic acid under conditions sufficient for a loop-mediated isothermal amplification, i.e. LAMP, of the SARS nucleic acid, wherein the set of LAMP primers comprises six primers of SEQ ID NOs: 1-7, thereby producing a SARS amplification sample;
- carrying out the LAMP from the SARS amplification sample, thereby producing a SARS amplification product; and
- detecting the SARS amplification product, thereby detecting the presence of the SARS nucleic acid in the sample.

## Description

The invention relates to a method and kits for detecting a viral infection, in particular for detecting coronavirus infections.

Over the past several decades, humankind has been confronted with a plethora of infectious pathogens that present differential diagnostic challenges due to similar on-set clinical symptoms and lead to diseases that afflict millions of people globally.

This was significantly the case since the end of 2019 when the SARS-CoV-2 virus has emerged.

Humanity was, after many years, confronted to a pandemic, and scientists and researchers have indented to rapidly and efficiently propose therapy, vaccine and diagnosis tests in order to eradicate or at least detect infections.

Diagnosis of SARS-CoV-2 infection is a key step in trying to contain the pandemic. Indeed, by detecting the infection of individuals, it is possible to implement isolation and confirmation strategies in order to limit contamination and therefore the spread of the virus.

At the beginning of the pandemic, PCR detection tests were rapidly carried out. However, these tests, although their effectiveness is acknowledged, are long and do not provide a rapid result allowing the implementation of emergency health measures.

More rapid tests have been therefore proposed to obviate these drawbacks.

Nucleic acid sequence based amplification or NASBA utilizes three enzymes to amplify a RNA product isothermally at 41 °C. A first primer containing a T7 promoter hybridizes to a target RNA and is extended by a reverse transcriptase (RT). RNase H then degrades the hybridized RNA to leave the bare cDNA. Next, a second primer hybridizes to the cDNA and is extended by the RT to the end of the initial hybridizing primer, producing a dsDNA containing a T7 promoter. T7 RNA polymerase then transcribes a RNA encoded between the regions where the primers originally annealed used. As multiple copies of RNA are made, free primer can continue to hybridize, be extended, and produce more template. This results in exponential amplification of the DNA template and RNA product.

Rolling circle amplification (RCA) involves a DNA or RNA polymerase that uses a circular DNA template to generate long RNA/DNA products. The circular template typically contains a polymerase promoter, hybridization sites, and template for a product that can act as a reporter (commonly a target site for a hybridization-based reporter, such as a molecular beacon). Unlike transcription with a linear target which produces a single copy of product, the polymerase can complete full circles of the circular template producing many copies. A method for exponential amplification involves hybridization oligonucleotides hybridizing to a target sequence, their ligation to form a closed circular template, and multiple copy production by a polymerase, the newly generated product containing multiple copies of the target sequence, which can act as new templates for linear template hybridization.

Loop mediated isothermal amplification (LAMP) utilizes two or three sets of primers with a strand displacing DNA polymerase to isothermally produce multiple mixed species of DNA product isothermally at 60-65°C. This method relies on producing DNA products containing single-stranded loop regions that allow for hybridization of primers to an already extended DNA product. This method, due to its very simple use was very largely developed for detecting SARS-CoV-2 infection in patients.

Some target genes are used in order to detect the presence of the virus in an individual. N gene and S gene (which code for the Spike protein) are frequently used. To date, many RT-LAMP diagnosis method are proposed in the worldwide market.

However, the experience of more than a year of pandemic has shown that the virus is able to mutate rapidly, and to emerge many genomic variants.

It is disclosed to date at least 5 variants of the virus (Variants of Concern), with different infectivity and contagiousness. Unfortunately, since mutations may occur in the sequences of the detection kit target sequence, many of them can fail to detect new variant and therefore the infection of a patient.

In view of this genetic variation, it is now absolutely mandatory to propose a diagnosis test which is at the same time rapid, efficient, which limits false positives, and more particularly which is able to detect the virus whatever the variant considered.

The invention therefore proposes to provide a method making it possible to carry out such tests.

The invention also provides a kit making it possible to implement such a method.

Another object of the invention is also to provide the use of this kit for a rapid and efficient diagnosis of the COVID-19 infection.

The invention therefore relates to a method of detecting, preferably in vitro, the presence of a SARS coronavirus nucleic acid in a biological sample, the method comprising:
- contacting the biological sample, with a sample buffer comprising at least a set of loop-mediated isothermal amplification primers specific for the SARS nucleic acid under conditions sufficient for a loop-mediated isothermal amplification, i.e. LAMP, of the SARS nucleic acid, wherein the set of LAMP primers comprises six primers:
- the six primers comprising or consisting of the nucleic acid sequences of SEQ ID NOs: 1-6; or
- the six primers comprising or consisting of the variant nucleic acid sequences having at least 90% sequence identity to any one of SEQ ID NOs: 1-6, provided that variant nucleic acid sequences allow to carry out the LAMP;
thereby producing a SARS amplification sample;
- carrying out the LAMP from the SARS amplification sample, thereby producing a SARS amplification product; and
- detecting the SARS amplification product, thereby detecting the presence of the SARS nucleic acid in the sample.

The invention is based on the unexpected observation made by the inventors that a specific set of primers allowing the amplification of a region of interest of the S gene can be associated with primers allowing to detect the amplification of another region of the genome of the virus, i.e. in another gene of the virus.

Loop-mediated isothermal amplification (LAMP) is a nucleic acid amplification technique that is primarily used for diagnosis and detection of diseases. It is a powerful amplification method that is carried out in an isothermal setting, compared to PCR which requires changes in the temperature during the amplification process. Amplification and detection of the nucleic acid could be completed at the same time, in a single step, by incubating the nucleic acid sample, 4 or 6 specifically designed primers, and Bst DNA polymerase in the same test tube at about 60 to 65 °C, depending on the optimal LAMP temperature.

The LAMP amplification process starts with the FIP complexing with the target nucleic acid at its F2 region to form double-stranded DNA, in equilibrium at around 65°C. The DNA polymerase with strand displacement activity then initiates DNA synthesis from the FIP, simultaneously displacing a single strand of DNA, if any. After this initiation step, the F3 primer then binds to its complementary F3c region and displaces the FIP-complementary strand. Because of the F1c sequence in the FIP, the FIP strand is able to self-anneal and form a loop structure at one end of the DNA. This strand then serves as the target for BIP-initiated DNA synthesis and subsequent strand displacement from B3-primed DNA synthesis. This allows the other end of the single DNA strand to form a loop structure, thus resulting in a dumbbell-like DNA structure. It serves as the template for subsequent amplification.

After a dumbbell-like structure formed, exponential amplification of the dumbbell structure is then initiated, with the DNA polymerase starting DNA synthesis at the F1 region. FIP also hybridizes to the one-loop structure from the F2 region, and DNA synthesis of that primer cause the F1-primed strand to be displaced and self-bind into a loop structure. Finally, from the B1 region in the new loop, self-primed DNA synthesis is again started, amplifying the current template while also creating a new one from the displacement of FIP-complementary strand. From this repeating process, it is possible to carry out a massive amplification, since DNA can be amplified as much as 10⁹ times during one hour. In LAMP with loop primers, six primers are used instead of four primers, with the forward and backward loop primers (LF and LB) annealing to regions between the F1/F2 and B1/B2 regions, respectively. The loop primers are proven to enhance the LAMP process, as amplification is fastened from the increased starting points for DNA synthesis. The LAMP method carried out in the invention is based on the use of 6+ primers, i.e. 6 primers commonly used, plus one specific FIP primer.

To extend LAMP from DNA diagnosis to RNA diagnosis as well as to realize multiplexed detection, several improvements were made to the original LAMP protocol in order to develop different methods, including reverse transcription LAMP (RT-LAMP) and multiplex LAMP. In RT-LAMP, RNA sequences are detected. This is particularly appropriated for developing a diagnosis method intending to detect the presence of a RNA virus. Reverse transcriptase is added to the LAMP mixture, to help in the conversion of viral RNA into complementary DNA (cDNA) that will be used for amplification according to the above-described technic.

In the invention the specific primers designed by the inventors are the following ones, and are complementary of specific sequences of the S gene of SARS-CoV-2 virus:

| | | |
|---|---|---|
| F3-Spir(21678->21693) | CTGACAAAGTTTTCAG | SEQ ID NO: 1 |
| B3-Spir(21867<-21885) | GTACCAAAAATCCAGCCTC | SEQ ID NO: 2 |
| FIP-Spir((21753<-21777(F1c) 21712->21732 (F2)) | CCAGAGACATGTATAGCATGGAAC CAACTCAGGACTTGTTCTTACC | SEQ ID NO: 3 |
| BIP-Spir((21778 - >21802(B1c)+21839<-21858 (B2)) | GACCAATGGTACTAAGAGGTTTGA TTTAGACTTCTCAGTGGAAGC | SEQ ID NO: 4 |
| LF-Spir House - 2(21734<-21751) | AGTAACATTGGAAAAGAA | SEQ ID NO: 5 |
| LB-Spir (21803 -> 21827) | AACCCTGTCCTACCATTTAATGATG | SEQ ID NO: 6 |

The bold text corresponds to a deletion of 6 bases in the FIP primer of the British variant, and to a SNP (C instead of A) in the BIP primer of the South-African variant.

Multiplexed LAMP assays have been developed in order to detect multiple pathogens in the same test tube, with the use of more primers or with unique fluorescent signals, and in particular for detecting different genes of the SARS-CoV-2 genome.

However, duplexing is not easy to carry out, 12 primers are needed to amplify DNA simultaneously in the same tube, without too much interactions. Duplexing was attempted with several other sets of primers, chosen from other areas of the genome, but without success, apart from the duplexing of two independently published primers systems. However, the annealing results are even less easy to interpret because the temperatures are closer. Fortunately, the design of the 6 primers as set forth in SEQ ID NO: 1-6 is such as the annealing results are easy to interpret since the temperatures between the two genes detected, or more, are significantly different.

In the invention, it is therefore possible to use only the 6 primers as listed above, but also additional primers located in another gene such as the N gene, or another gene of the virus, such as ORF1ab or E genes.

It is advantageous that both S and N genes be amplified in the method according to the invention.

Advantageously, the invention relates to the method as defined above, comprising prior to carrying out the LAMP from the SARS amplification sample,
- contacting the biological sample with a preparing buffer, in order to obtain an inactivation mixture, and
- contacting the inactivation mixture with a sample buffer comprising a set of loop-mediated isothermal amplification primers specific for the SARS nucleic acid under conditions sufficient for a loop-mediated isothermal amplification, i.e. LAMP, of the SARS nucleic acid.

In other words, the invention relates to a method of detecting, preferably in vitro, the presence of a SARS coronavirus nucleic acid in a biological sample, the method comprising:
- contacting the biological sample with a preparing buffer, in order to obtain an inactivation mixture,
- contacting the inactivation mixture with a sample buffer comprising at least a set of loop-mediated isothermal amplification primers specific for the SARS nucleic acid under conditions sufficient for a loop-mediated isothermal amplification, i.e. LAMP, of the SARS nucleic acid, wherein the set of LAMP primers comprises six primers:
- the six primers comprising or consisting of the nucleic acid sequences of SEQ ID NOs: 1-6; or
- the six primers comprising or consisting of the variant nucleic acid sequences having at least 90% sequence identity to any one of SEQ ID NOs: 1-6, provided that variant nucleic acid sequences allow to carry out the LAMP;
thereby producing a SARS amplification sample;
- carrying out the LAMP from the SARS amplification sample, thereby producing a SARS amplification product; and
- detecting the SARS amplification product, thereby detecting the presence of the SARS nucleic acid in the sample.

Advantageously, the invention relates to the method defined above, wherein said SARS coronavirus, is a SARS-CoV-2, or one of its variants of concern, such as a British variant, South-African variant, Brazilian variant, Indian variant, or any other variant.

In the invention, the "wild-type" virus corresponds to the original strain isolated in Wuhan, China, in 2019. The genome of the virus is entered in Genbank database under the reference number MN908947.3.

In the invention, when referring to SARS-CoV-2, reference is made to this "wild-type" virus, having the sequence as set forth in SEQ ID NO: 15.

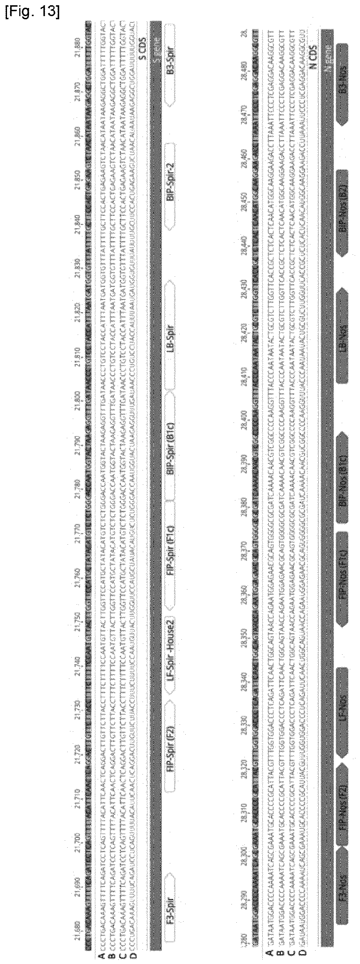

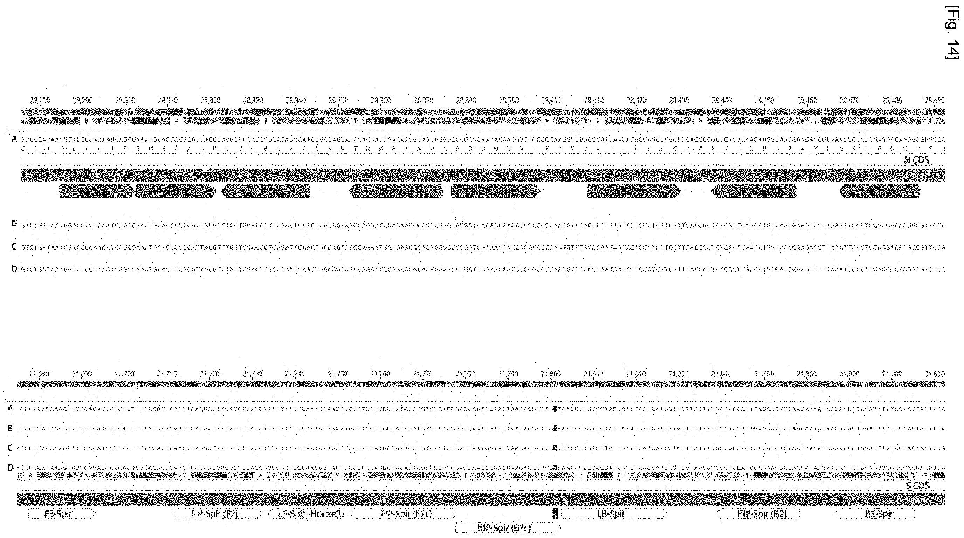

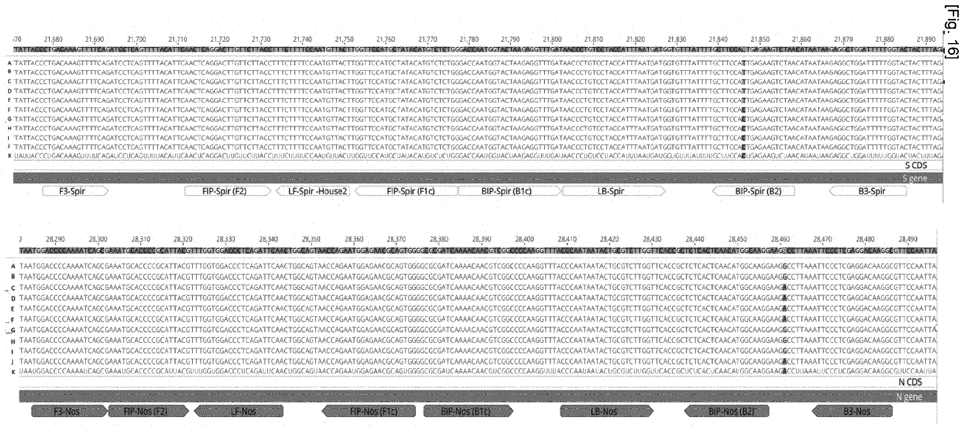

Figures 13, 14, 15 and 16 represent the positioning of the primers used in the invention in SARS-CoV-2 genomes of Brazilian.

Advantageously, the invention relates to the method defined above, primers comprising 7 primers as set forth in SEQ ID NO: 1-7 or SEQ ID NO: 1-6 and 14.

Advantageously, the invention relates to the method defined above, the method comprising:
- contacting the biological sample with a sample buffer comprising at least a set of loop-mediated isothermal amplification primers specific for the SARS nucleic acid under conditions sufficient for a loop-mediated isothermal amplification, i.e. LAMP, of the SARS nucleic acid, wherein the set of LAMP primers comprises seven primers:
- the seven primers comprising or consisting of the nucleic acid sequences of SEQ ID NO: 1-7 or SEQ ID NO: 1-6 and 14; or
- the seven primers comprising or consisting of the variant nucleic acid sequences having at least 90% sequence identity to any one of SEQ ID NO: 1-7 or SEQ ID NO: 1-6 and 14, provided that variant nucleic acid sequences allow to carry out the LAMP;
thereby producing a SARS amplification sample;
- carrying out the LAMP from the SARS amplification sample, thereby producing a SARS amplification product; and
- detecting the SARS amplification product, thereby detecting the presence of the SARS nucleic acid in the sample.

Advantageously, the invention relates to the method defined above, the method comprising:
- contacting the biological sample with a preparing buffer, in order to obtain an inactivation mixture,
- contacting the inactivation mixture with a sample buffer comprising at least a set of loop-mediated isothermal amplification primers specific for the SARS nucleic acid under conditions sufficient for a loop-mediated isothermal amplification, i.e. LAMP, of the SARS nucleic acid, wherein the set of LAMP primers comprises seven primers:
- the seven primers comprising or consisting of the nucleic acid sequences of SEQ ID NOs: 1-7 or SEQ ID NO: 1-6 and 14; or
- the seven primers comprising or consisting of the variant nucleic acid sequences having at least 90% sequence identity to any one of SEQ ID NO: 1-7 or SEQ ID NO: 1-6 and 14, provided that variant nucleic acid sequences allow to carry out the LAMP;
thereby producing a SARS amplification sample;
- carrying out the LAMP from the SARS amplification sample, thereby producing a SARS amplification product; and
- detecting the SARS amplification product, thereby detecting the presence of the SARS nucleic acid in the sample.

The inventors have also made the unexpected observation that:
a) RT-LAMP can be carried out by using 7 primers that can both detect Sars-CoV-2 variant having either or not a deletion in the gene S (deletion that is observed in the British variant); and
b) that two nucleotides covering an overlapping region can be used simultaneously for DNA amplification when carrying out a RT-LAMP.

The observation made by the inventors is that when using primers targeting the S gene of the virus SARS-CoV-2, depending upon the design of the primers, some of them cannot be able to hybridize with their target due to a deletion, e.g. the deletion that occurs in the S gene of the British variant. In order to overcome this drawback, the inventors have designed 2 oligonucleotides that cover the same region within the sequence of the gene S, one being complementary to the "wild type sequence", the other one being complementary to the "deleted sequence", i.e. the sequence that is found in the British variant.

Particularly, the inventors designed the 3 following FIP-Spir primers:

| | | |
|---|---|---|
| FIP-Spir((21753<-21777(F1c) 21712->21732 (F2)) | CCAGAGACATGTATAGCATGGAACCAA CTCAGGACTTGTTCTTACC | SEQ ID NO: 3 |
| FIP-Spir-UK1 | CCAGAGATAGCATGGAACCAAACTCAG GACTTGTTCTTACC | SEQ ID NO: 7 |
| FIP-Spir-UK2 | CCAGATATAGCATGGAACCAAACTCAG GACTTGTTCTTACC | SEQ ID NO: 14 |

Surprisingly, and advantageously, both primers can be used simultaneously, with the other primers in order to carry out the LAMP reaction, and to give relevant results.

The originality of the oligonucleotides designed by the inventors is that the primer FIP-Spir is complementary to the sequence of the S gene, but covers the deletion that was observed in the British variant. Therefore, the inventors proposed an additional primer FIP-Spir-UK, the sequence of which does not contain the nucleotides deleted in the British variant.

It is also noticeable that the primer BIP-Spir mentioned above contain the nucleotide (bold underlined) which is mutated in the South-African variant. However, this single mutation in this specific variant does not affect the efficacy of the method, and the LAMP can be carried out.

Advantageously, the invention relates to the method as defined above, wherein the set of LAMP primers comprises 13 primers comprising or consisting of the nucleic acid sequences of SEQ ID NOs: 1-13 or SEQ ID NO: 1-6 and SEQ ID NO: 8-14.

The inventors have also identified that the method according to the invention is advantageously efficient to detect SARS-CoV-2 presence in a biological sample, or one of its variants, when carrying out a RT-LAMP by using simultaneously primers allowing the amplification of both S and N genes of the virus.

As mentioned above, the inventors show that it is possible to carry out a LAMP method by using simultaneously primers allowing the amplification of both S and N genes, without interference or cross-reaction.

In the invention, the specific primers designed by the inventors are the following ones, and are complementary of specific sequences of the N gene of SARS-CoV-2 virus:

| | | |
|---|---|---|
| F3-Nos(28285 -> 28302) | TGGACCCCAAAATCAGCG | SEQ ID NO: 8 |
| B3-Nos(28468 <-28486) | GCCTTGTCCTCGAGGGAAT | SEQ ID NO: 9 |
| FIP-Nos((28353 <-28374(F1c) +28303-> 28321 (F2)) | CCACTGCGTTCTCCATTCTGGTAAATGCA CCCCGCATTACG | SEQ ID NO: 10 |
| BIP-Nos((28377 - > 28397(B1c) + 28438 <-28457(B2) | CGCGATCAAAACAACGTCGGCCCTTGCC ATGTTGAGTGAGA | SEQ ID NO: 11 |
| LF-Nos(28323 <-28343) | TTGAATCTGAGGGTCCACCAA | SEQ ID NO: 12 |
| LB-Nos(28409 -> 28430) | CCCAATAATACTGCGTCTTGGT | SEQ ID NO: 13 |

The protocol used in the invention will be better detailed in the Example below.

It can be noted however that the annealing temperature for the amplification corresponding to the S gene about 84°C +/- 1.5°C and the annealing temperature for the amplification corresponding to the N gene about 89°C +/- 2°C. This temperature difference is significantly important to allow a separated detection of both amplification within the same screen. Therefore, the method according to the invention is appropriate for the detection, at the same time, of the presence of the S and N genes of SARS-CoV-2 virus, and also its variants.

When carrying out the method according to the invention, the biological sample should be:
- obtained from the patient, or the individual suspected to be infected by the virus,
- then placed
   a- either directly in contact with the sample buffer containing the primers as defined above, i.e. containing the primer as set forth in SEQ ID NO: 1-6, preferably as set forth in SEQ ID NO: 1-7 or SEQ ID NO: 1-6 and 14, more preferably as set forth in SEQ ID NO: 1-13 or SEQ ID NO: 1-6 and SEQID NO: 8-14, or
   b- in contact with a preparing buffer or, i.e. a buffer allowing to denaturate/inactivate the virus contained in the biological sample and inactivate any undesirable enzymes (such as RNAse or DNAse), prior to be placed in contact with the sample buffer,
- and then completed with enzymes necessary for carrying out the LAMP reaction (i.e. Reverse transcriptase, DNA polymerase...).

More advantageously, the method can be carried out as defined above, wherein the sample buffer contains both the primers and the enzymes necessary for carrying out the LAMP reaction, along with any compounds necessary for the reactions such as salts, nucleotides ...).

More advantageously, the invention relates to the method as defined above, wherein the sample buffer is lyophilised.

Lyophilization of the sample buffer is used for allowing the stability of the sample buffer, and to enhance its life storage. Moreover, lyophilizing the sample buffer can also favor the volume adjustment when preparing the sample for carrying out the LAMP reaction.

Therefore, the biological sample, for instance a nasal swab sample, can be added to a tube into which the sample buffer is lyophilized at the bottom. Then a standardized volume of RNase free water can be added into the tube to allow the contact between the virus liable to be present in the sample and the primers. Then enzymes can be added (DNA polymerase).

When a preparing buffer is used, i.e. when it is advantageous to favor the condition for carrying out the LAMP reaction, this preparing buffer can be lyophilized. However, in this case, it is advantageous that the sample buffer be not lyophilized in order to avoid any contamination when transferring the mixture of the biological sample and the preparing buffer into the tube containing the sample buffer.

In this case, the biological sample is contacted with the preparing buffer, which is lyophilized in a tube, and an appropriated volume of water (preferably RNAse free water) is added. After incubation, the sample buffer is added to the tube, and possibly, if they are not already contained in the sample buffer, enzymes and nucleotides are also added. Then the tube is ready for use to carry out the LAMP reaction.

More advantageously, the invention relates to the method as defined above, wherein the inactivation mixture is heated at 95°C, preferably during 3 to 15 min, more preferably about 5 min.

When using a biological sample containing, or liable to contain, a native virus, the nucleic acid molecule of the virus is contained within the envelop. Therefore, the primers have not a direct access to the viral genome. Moreover, when preparing the sample, it is frequent that the sample be contaminated by enzymes that could interfere with the lamp reaction, such as DNAse, RNAse... It is therefore advantageous to carry out a step of inactivation of these enzymes in order to favor the LAMP reaction. Heat inactivation during a short time is particularly advantageous, such as 95°C for less than 15 min, preferably from 5 to 10 min, more preferably for 5 min.

It is also possible to add in the sample buffer chelating agents such as EDTA, EGTA or the likes, and inhibitors of DNAse or RNAse. A combination of chemical inhibitors (chelating agents and DNAse and RNAse) and heat inactivation is more advantageous.

Advantageously, the invention relates to the method described above, further comprising contacting the SARS amplification sample with a reverse transcriptase under conditions sufficient for reverse transcription of the viral nucleic acid.

When the sample contains a native virus, the nucleic acid target of the primer necessary to carry out the LAMP reaction is an RNA. Therefore, it is mandatory to reverse-transcript this RNA into a complementary DNA before processing to the LAMP reaction.

Therefore, transcriptase inverse should be added in the reaction sample.

Advantageously, the invention relates to the method described above, wherein detecting the viral nucleic acid amplification product comprises the use of fluorescence detection.

In order to detect the synthesis of the double stranded DNA during the LAMP reaction, fluorescent dyes can be used. The LAMP fluorescent dyes are intercalating dyes that can be used to track a LAMP reaction in real-time by fluorescence measurement. SYTO-42, SYTO-82, SYTO-84, SYTOX Orange and SYTO-81 can be used and have the widest range of working concentrations. More generally, any fluorescent dye that can intercalate between the two strands of a double stranded DNA can be used. The skilled person will choose the more suitable one.

More advantageously, the invention relates to the method as defined above, wherein the sample comprises isolated DNA, isolated RNA, blood, plasma, serum, feces, saliva, nasopharyngeal sample.

Detection and quantification of SARS-CoV-2 can be carried out by the method according to the invention. Depending upon the sample to be tested, the conditions can vary.

For biological samples containing free DNA corresponding to the genome of the virus, it is not mandatory to carry out a step of reverse transcription. The primer can hybridize directly with their target, and the amplification can be achieved.

When using a biological sample containing, or liable to contain, a native virus, a reverse transcription reaction could be advantageous. These kinds of biological are blood, plasma, serum, feces, saliva, nasopharyngeal sample. These biological samples can also encompass wastewaters obtain from house canalizations.

In another aspect, the invention relates to a composition comprising at least one set of LAMP primers specific for a SARS nucleic acid, the set comprising six primers comprising or consisting of the nucleic acid sequences of SEQ ID NOs: 1 to 6.

In another advantageous aspect, the above-mentioned composition may contain an additional primer specific for the SARS nucleic acid, the primer comprising or consisting of the nucleic acid sequences of SEQ ID NO: 7 or 14.

More advantageously, the above-mentioned composition further comprising six primers comprising or consisting of the nucleic acid sequences of SEQ ID NOs: 8 to 13.

In other words, the invention relates to a composition comprising or consisting essentially of 13 primers as set forth in SEQ ID NO: 1-13 or SEQ ID NO: 1-6 and 8-14.

The composition as defined above can be completed by compounds that are necessary for carrying out a LAMP reaction, such as, salts, nucleotides, DNA polymerase, Reverse transcriptase... This list is not limitative.

The invention also relates to the use of the above-mentioned composition, for diagnosing the presence of a SARS virus in a sample.

Advantageously, the invention relates to the above-mentioned composition, for its use for diagnosing the presence of a SARS virus in a sample.

The above-mentioned composition, i.e. the primers allowing the amplification of a region of each of the S and N genes, can be used to diagnose infection by SARS-CoV-2 virus, or one of its variants, and to diagnose that an individual is affected by COVID-19 (Corona Virus 2019) disease.

In another aspect, the invention relates to a kit comprising:
- a set of LAMP primers specific for a coronavirus, in particular SARS-CoV-2 virus, nucleic acid comprising 7 primers comprising or consisting of the nucleic acid sequences of SEQ ID NOs: 1 to 7; and
- a preparing buffer.

This kit can be ready-to-use in order to implement LAMP reaction for the purpose of diagnosis of SARS-CoV-2 infection.

Advantageously, the primers solubilized in a sample buffer that contains salts that can be useful to i) place the replication enzyme in a favorable condition in order to catalyze replication of DNA, and/or ii) to inhibit enzymes or compounds liable to degrade RNA or DNA, or both, or liable to inhibit the DNA replication.

More advantageously, the kit according to the invention is such that
- the set of LAMP primers is in a lyophilized form, or
- the preparing buffer is in a lyophilized form.

This lyophilizate can be therefore resuspended in an aqueous solvent prior to carry out a LAMP reaction. The lyophilizate is advantageously placed at the bottom of a sample reaction tube.

In an advantageous kit, the kit comprises a tube having in its bottom a lyophilized set of LAMP primers, and another tube containing in a liquid form, or in a lyophilized form the preparing buffer. In this configuration of the kit, the biological sample will be placed in the tube containing the preparing buffer, and possible water is added. Then the content of the tube is placed onto the tube containing the LAMP primers before carrying out the LAMP reaction.

Another advantageous kit comprises a tube with a lyophilized preparing sample into which is placed the biological sample. Another tube contains the primers, but can also contains all the compounds necessary to carry out the enzyme reaction (reverse transcription, DNA replication).

The kit according to the invention is not limited to the above described forms, and the term "tube" should not be limited to a specific configuration, but designates any container that can be used for a biological purpose, in particular for a diagnosis purpose.

Advantageously, the invention relates to the above-mentioned kit, wherein the sample buffer comprises tris(2-carboxyethyl)phosphine and ethylenediaminetetraacetic acid.

Tris(2-carboxyethyl)phosphine is a reducing agent frequently used in biochemistry and molecular biology applications. This compound is an irreversible reducing agent that powerfully and efficiently denatures proteins, and therefore can inhibit DNAse and RNAse liable to be present in the biological sample used for carrying out the LAMP reaction.

Ethylenediaminetetraacetic acid is a chelating agent used to sequester metal ions in aqueous solution, and that deactivates metal-dependent enzymes such as DNAse and RNAse.

The invention will be better understood from the teaching of the following examples, illustrated by the following figures.

### Brief description of the drawings

[Fig. 1] **Figure 1** represents the different steps of the method according to the invention. a) collection of biological samples (naso-pharyngeal swabs); b) addition to the samples of 1% inactivation solution and heating at 95°C for 5 min; c) addition to the inactivated RNA extract of primers and enzymes; and d) RT-LAMP reaction in OptiGene Genie II device: amplification 25 min and annealing 5 min.
[Fig. 2] **Figure 2** represents a graph showing the fluorescence intensity during the time of LAMP reaction (x-axis: time in hh:mm:ss). A: inactivated clinical sample 1; B: inactivated clinical sample 2; C: inactivated clinical sample 3; D: negative control 1; E: inactivated clinical sample 4; F: inactivated clinical sample 5; G: positive control and H: negative control 2.
[Fig. 3] **Figure 3** represents a graph showing the annealing temperatures (Ta, equivalent of Tm) generated by a melting curve analysis of RT-duplex LAMP assay. (x-axis: temperature in Celsius; y-axis: fluorescence). Two target sequences (S and N) are amplified during LAMP reaction.

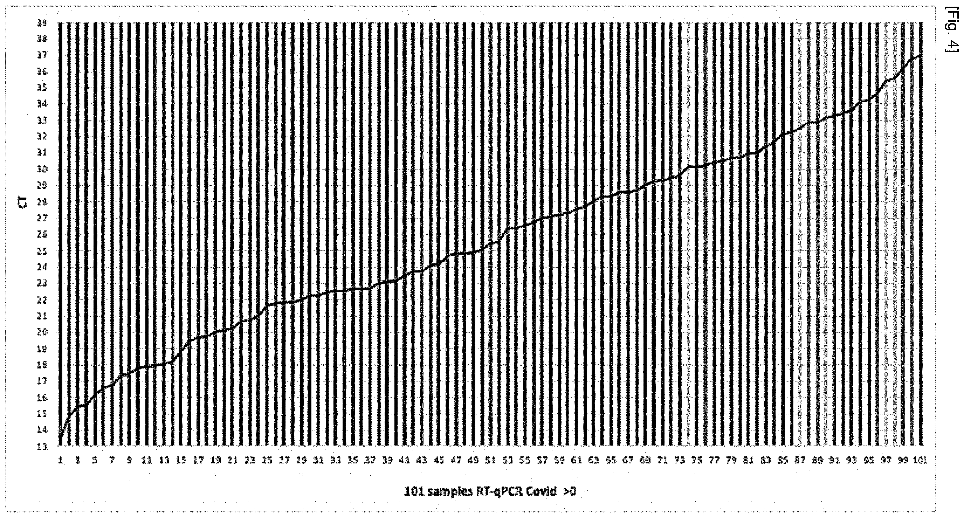

[Fig. 4] **Figure 4** represents a graph showing the correlation between the qPCR and the RT-LAMP responses obtained on 101 clinical positive samples. (x-axis: Ct values. y-axis: the 101 clinical samples ordered according to their Ct values, from the lowest to the highest). The dark curve corresponds to the Ct values of the different samples. The corresponding RT-LAMP response of each sample is indicated by a vertical bar: dark for a positive response (2/2 replicates tested positive), light colored for an intermediate response (1/2 replicate tested positive) or a negative response (0/2 replicate tested positive).
[Fig. 5] **Figure 5** represents a graph showing the temperature profile of the experiment.
[Fig. 6] **Figure 6** represents a graph showing the amplification curve obtained for the UK variant (grey) and the positive control (black) (400 copies of synthetic genome Sars-Cov-2 ref GenBank: MN908947.3, Imagene).
[Fig. 7] **Figure 7** represents a graph showing the annealing curves for the two samples. For the UK sample (grey), the spike peak is missing.
[Fig. 8] **Figure 8** represents a graph showing the temperature profile of the experiment.
[Fig. 9] **Figure 9** represents a graph showing the amplification curve obtained for the South African variant (grey) and the positive control (black) (400 copies of synthetic genome Sars-Cov-2 ref GenBank: MN908947.3, Imagene).
[Fig. 10] **Figure 10** represents a graph showing the annealing curves for the two samples. D Expression of results: time peak values (= time to results) are given for the two samples.
[Fig. 11] **Figure 11** represents graph showing the annealing curves for eight samples of British variant and a negative control.
[Fig. 12] **Figure 12** represents graph showing the annealing curves for eight samples of original strain of SARS-CoV-2 and a negative control.
[Fig. 13] **Figure 13**: Some randomly chosen sequences of Brazilian variant (A, B and C) from the Gisaid database were aligned with the Sars Cov-2 reference sequence NC_045512 (D). The primers according to the invention are positioned for both regions corresponding to S and N genes.
[Fig. 14] **Figure 14****:** Some randomly chosen sequences of South African variants (left panel: B-D and right panel: A-C) from the Gisaid database were aligned with the Sars Cov-2 reference sequence NC_045512 (left panel: A and right panel D). No mutation was found for Nos region. The primers according to the invention are positioned for both regions corresponding to S and N genes. For Spir region a SNP (C instead of A) was found at the 3' extremity of the B1c part of BIP primer.

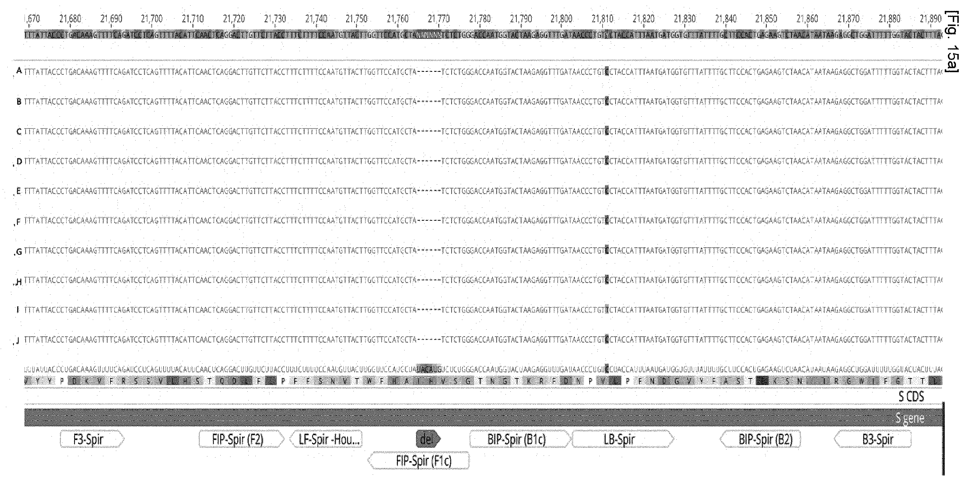

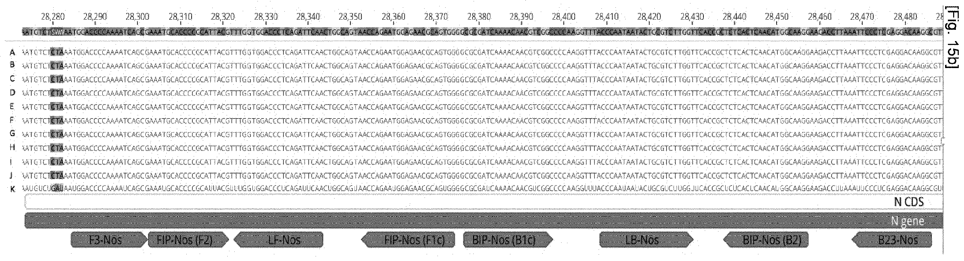

[Fig. 15a] and [Fig. 15g] **Figure 15a** **and** **15b**: Some randomly chosen British variant sequences (Fig. 15a: A-I; Fig. A-J) 15b: from the Gisaid database were aligned with the Sars Cov-2 reference sequence NC_045512. The primers according to the invention are positioned for both regions corresponding to S and N genes. For Spir region of the UK variants there was a deletion of 6 bases corresponding to the F1c part of FIP-Spir. For the 9th sequence of British variant a T replaced a C in the internal part of the LB-Spir. Nevertheless, it can be due to an error of sequencing. Anyway, according to the location of the mutation (internal) and the nature of the mismatch (C:T), this mutation have no effect on the LAMP reaction.
[Fig. 16] **Figure 16**: Some randomly chosen sequences of Indian variants (A-J) from the Gisaid database were aligned with the Sars Cov-2 reference sequence NC_045512 (K). The primers according to the invention are positioned for both regions corresponding to S and N genes. A SNP (T instead of G) was detected for less than half of the variants. Nevertheless, according to the location of the mutation (internal) and the nature of the mismatch (C:T), this mutation should have no effect on the LAMP reaction.

### Examples

### Example 1- Primers and preparations

Mix of primers are prepared as follows as separated 10X concentrated primers:
Mix#1 (Spir) - 100µL

**[Table 4]**

| | Initial concentration µM | Final concentration µM (in LAMP reactional mix) | Volume µL |
|---|---|---|---|
| FIP-Spir | 100 | 1.2 | 12 |
| BIP-Spir | 100 | 1.2 | 12 |
| LF -Spir House 2 | 100 | 0.6 | 6 |
| LB-Spir | 100 | 0.6 | 6 |
| F3-Spir | 100 | 0.3 | 3 |
| B3-Spir | 100 | 0.3 | 3 |
| H₂O | | | 58 |

Mix#2 (Nos) - 100µL

**[Table 5]**

| | Initial concentration µM | Final concentration In LAMP reactional mix) µM | Volume µL |
|---|---|---|---|
| FIP-Nos | 100 | 0.8 | 8 |
| BIP-Nos | 100 | 0.8 | 8 |
| LF -Nos | 100 | 0.4 | 4 |
| LB-Nos | 100 | 0.4 | 4 |
| F3-Nos | 100 | 0.2 | 2 |
| B3-Nos | 100 | 0.2 | 2 |
| H₂O | | | 72 |

**Mix SPIR** - MiX#1-1 containing 7 primers - 100 µL

**[Table 6]**

| | Initial concentration µM | Final concentration µM | Volume µL |
|---|---|---|---|
| FIP-Spir | 100 | 1.2 | 12 |
| BIP-Spir | 100 | 1.2 | 12 |
| FIP-Spir-UK1 | 100 | 0.6 | 6 |
| LF -Spir House 2 | 100 | 0.6 | 6 |
| LB-Spir | 100 | 0.6 | 6 |
| F3-Spir | 100 | 0.3 | 3 |
| B3-Spir | 100 | 0.3 | 3 |
| H₂O | | | 52 |

For carrying out the LAMP reaction the following reactive mix is prepared:
per well are added:
- 30 µL of Master mix RT ISO 004 (Optigene)
- 4,95µL of Mix#1 or Mix#1-1
- 4.95µL of Mix#2
- 0.1µL AMV Reverse transcriptase (1 U final concentration)
- 10µL of biological sample (further to inactivation),

It is also possible to prepare a reaction mix of 25µL, in which all the volumes are divided by 2.

### Preparation of the inactivation sample (100X - EDTA - 100mM + TCEP 250mM)

Weigh 358 mg of TCEP then dissolve it in 3.5mL of water, add 186mg of EDTA then adjust the pH to obtain a neutral pH (7.0). To obtain a pH -7.0, dissolve the solution and add 500µl of 10N NaOH in the 3.5ml of solution.

Once the pH has been adjusted, make up the volume with reverse osmosis water to obtain 5ml of 100X inactivating solution.

This solution can be stored for 1 month at 4°C.

Dilution of the inactivation solution to 2X (ST1 2X):
Dilute the 100x inactivation solution 50 times in RNAse free water.

Preparation of strips containing 2X ST1 solution and lyophilization
- Depending on the lyophilizer used, heat the pump beforehand (duration: 20min)
- Distribute 5µl of 2X ST1 solution in each well of the strips and leave the wells opened.
- Paste a strip of parafilm on all the wells of a strip, and then use a needle to make a small hole for each well.
- Centrifuge each strip before starting lyophilization
- Carry out the lyophilization about 30min in small appliances (pression 0.05mbar and temperature -55°C).
- Once lyophilization is completed, remove the parafilm on each strip and close the wells.
- Store the strips in a dry place at room temperature and protected from light.

Precautions to be taken: Decontaminate surfaces with RNAse away, decontaminate consumables (strips, racks, tubes) under UV, for solution preparation: use sterile utensils as much as possible.

### Example 2- A rapid and sensitive real-time duplex RT-LAMP for detection of Sars-CoV2 on site

Most of the LAMP protocols developed for SarsCov-2 are simplex assays, amplifying only one target in a single test. In the present invention, the inventors developed a rapid real-time duplex RT-LAMP assay named RUNCOV, able to detect SARS-CoV-2 with a high sensitivity level without RNA extraction step. RUNCOV targets two different SARS-CoV-2 genes: S and N genes, encoding for spike and nucleocapsid proteins, respectively. A rapid RNA preparation step was implemented for analyzing biological samples, in order to inactivate the virus prior to the RT-LAMP assay. This protocol was successfully applied on clinical diagnostic swabs of human patients. RUNCOV is currently involved in several Point of Care Testing (POCT) programs, such as setting up tests at the University, at the hospital entrance or at the airport.

### Materiel and methods

### Analytical specificity

RUNCOV protocol was tested on RNA from several nontarget viruses: other respiratory viruses inducing similar clinical signs and some coronaviruses isolated from animals (bats, pig and poultry) (see Table 1 below).

### Analytical sensitivity

Analytical sensitivity of RUNCOV was evaluated on ten-fold dilutions series in pure water of RNAshell^{®} minicapsules of synthetic genome Sars-Cov-2 (Genebank: MN908947.3) (Imagene, Ivry, France) or in negative swab sample Amies (Greiner).

RUNCOV was also performed on three clinical samples serially diluted in pooled negative swab samples in Amies (Greiner) or in Microtest M4RT (Remel). RNA was extracted from the different dilutions and tested with RT-qPCR assay according to the protocols described in below.

### Tests on Clinical Samples

Two hundred and four nasopharyngeal swabs were provided by Biological Resource Center (BRC) of the "Centre Hospitalier Universitaire de la Reunion (CHU) Felix Guyon" and or by a private laboratory (REUNILAB). The samples were collected from patients and placed into sterile tubes containing Amies or PBS Transport medium (Greiner). Among these samples, 101 were tested positive and 103 were tested negative with a Sars-CoV2-specific real-time quantitative assay (see protocol below).

A trial was organized in collaboration with CHU La Reunion Sud (project "dépistage RUNCOV CHU SUD"), under the supervision of the BRC. The RT-LAMP was carried out on site, at the Covid screening center. The samples were collected in sterile tubes containing Amies. Sample preparation and RUNCOV assay were performed according to the protocol described below in § "Preparation of RNA samples and direct LAMP SARS-CoV-2 virus detection". Samples were also analyzed by qPCR according to the protocol below.

### RNA extraction and RT-qPCR SARS-CoV-2 detection

Samples were extracted using the NucleoSpin^{®} RNAVirus Kit (Macherey Nagel) and RT-qPCR were performed on Quantstudio 5 instrument (Thermofisher). Amplifications were performed on 2 µl extract using the superscript III Platinium mix (Invitrogen) and the primer/Taqman^{®} probe systems IP2 and IP4, according to the protocol developed by French referral laboratory Institut Pasteur Paris (https://www.who.int/docs/default-source/coronaviruse/real-time-rt-pcr-assays-for-the-detection-of-sars-cov-2-institut-pasteur-paris.pdf?sfvrsn=3662fcb6_2).

### Preparation of RNA samples and direct LAMP SARS-CoV-2 virus detection

Samples were treated with 1% of a 100X buffer lysis solution (250 mM TCEP, 100 mM EDTA, adjusted to pH7 with NaOH), then mixed and heated at 95°C for 5 minutes, as described in Rabe et al., 2020.

After sample treatment, RT-LAMP reactions were performed using a portable device (Genie II, Optigene, or any other similar device). RUNCOV reactions (i.e; LAMP reaction according to the invention) were performed in 50 µl total reaction volume, containing 30 µl ISO-DR004-RT300 Isothermal Mastermix (Optigene), 4,95 µl of primer mix N and 4,95 µl of primer mix S, in order to obtain final concentrations of 0.2 µM and of each F3-N and B3-N primers, 0.8 µM of each FIP-N and BIP-N primers, 0.4 µM of each loop-N primers, and 0.3 µM of each F3-S and B3-S primers, 1.2 µM of each FIP-S and BIP-S primers and 0.6 µM of each loop-S primers, 1 unit of AMV Reverse transcriptase (Promega) and 10 µL of template. LAMP reactions were run at 65°C for a period of 25 min followed by an anneal step with temperature varying from 92°C to 78°C at a speed of 0.05°C/second.

### Viral concentration in samples

Samples (1 ml) were centrifuged at 4000g for 10 minutes at room temperature, the pellet was resuspended in 20 µl of 1X buffer lysis solution, and heated at 95°C for 5 minutes.

For samples collected in complex media like Microtest M4RT (Remel), or UTM (Copan), a cleaning step of the pellet was implemented to remove inhibitors. For this, the pellet was first resuspended in PBS 1X pH 7.4 buffer and centrifuged at 4000g for 10 minutes, and further treated as described above.

### Viral load measurement over time

Nasopharyngeal samplings were performed over time on a patient showing classical symptoms of Covid-19. Swabs were collected in the Microtest M4RT medium (Remel) at J7, J10, J12 and J14 after the apparition of symptoms. Samples were tested with RUNCOV assay after the inactivation step, or were concentrated and cleaned by centrifugation as described above.

### Tests on variants.

The response of RUNCOV on the different variants: South Africa (501Y.V2), Brazil (B.1.1.248) and British (VOC 202012/0) was examined by an *in silico* analyzis using Geneious. The different known mutations were localized on the genomes and predictions of amplification by the two LAMP primers sets were performed according to the software Primer explorer guidelines (https://primerexplorer.jp/e/v5_manual/index.html).

Samples from UK (n=10: 6 clinical samples collected in Amies swabs, 2 RNAshell^{®} minicapsules of synthetic genome Sars-Cov-2 from Imagene (ref GenBank: EPI_ISL_710528), and 2 RNA extracted from Amies swabs) and South Africa variants (n=12: 10 clinical samples collected in Amies swab and 2 RNA extracts) were also tested with RUNCOV.

### RESULTS AND DISCUSSION

### RUNCOV: a rapid and portable Sars-Cov-2 specific duplex RT-LAMP assay

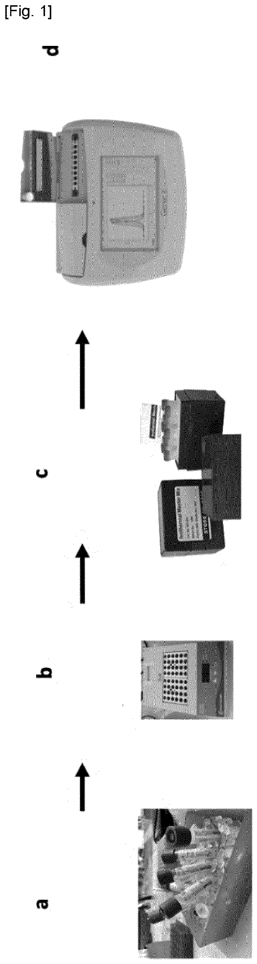

The inventors have developed a rapid RT-LAMP test based on real-time fluorescence monitoring and targeting two regions of the Sars-Cov2 genome, S and N. The reliability of the test is supported by a post -amplification step which allows to verify that the expected annealing temperature of the amplicons is obtained. The different steps of RUNCOV are detailed in **Figure 1**.

The test is carried out using nasopharyngeal swabs (a). The recommended sampling media are phosphate saline (PBS) or Amies transport medium. More complex media such as UTM or M4RT are not recommended. Nevertheless, if necessary, they can be used with an additional centrifugation step to remove inhibitory substances from the samples (see above § "Concentration/cleaning step and viral load measurement over time)".

The samples are treated with 1% inactivation solution and heated at 95°C (b). This quick step allows both to inactivate the virions ant to protect the viral RNA from RNAses. Indeed, TCEP (tris(2-carboxyethyl)phosphine) is able to reduce any disulfide bridges and to participate in the denaturation of the proteins. EDTA (ethylene diamine tetraacetic acid) is able to capture any divalent cations necessary for RNAse activity.

RNA sample is then added to a reactional mix including the MasterMix, the 12 LAMP primers (a 10X primer mix is constituted for each target) and AMV-Reverse transcriptase (efficient for templates with high secondary structure, as recommended by Optigene to improve the RT-LAMP reaction) (c). Amplification is run at 65°C during 25 min followed by a post-amplification step (5 min) (d).

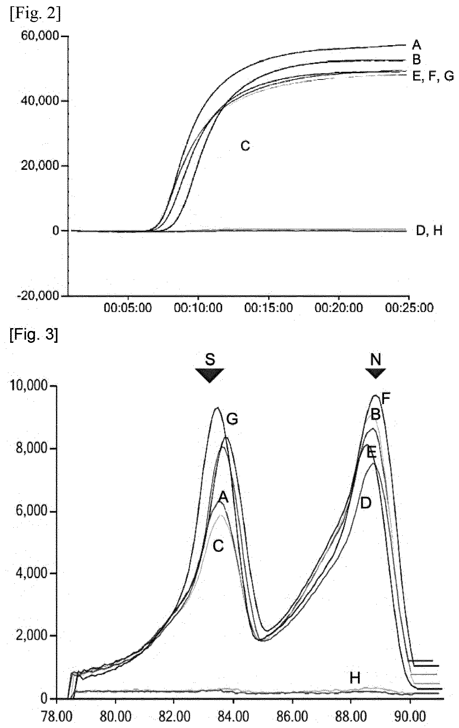

For each sample, a fluorescence measurement is performed over time (amplification curve) (**Figure 2**). A Ratio peak time (named peak value in table 1) is generated, reflecting the amplification take off time during the amplification phase (**Table 1**).

**[Table 1]**

| Well | Peak value (mm:ss) |
|---|---|
| A: AL gre clt i | 8:00 |
| B: AL gre clt i [2] | 8:00 |
| C: AL remel dir i | 10:15 |
| D: T- | |
| E: AL remel clt i | 8:30 |
| F: AL remel clt I [6] | 8:15 |
| G: T+ p1 -3 | 9:15 |
| H: T- [8] | |

This peak time is similar to the Ct or Cq values in qPCR assays. During the post-amplification phase, annealing peaks are generated (derivative of fluorescence plotted against temperature) which are signatures of the amplified products (**Figure 3** and **Table 2**). A sample will be considered positive if both a peak time value and at least one of the specific annealing temperatures (Ta) are obtained (83°C ≤ Ta ≤ 85.5°C for S and 87.5°C ≤ Ta ≤ 91°C for N).

**[Table 2]**

| Well | Peak value (°C) |
|---|---|
| A: AL gre clt i | 88.7 |
| B: AL gre clt i [2] | 88.6 |
| C: AL remel dir i | 88.7 |
| D: T- | |
| E: AL remel clt i | 83.6 |
| F: AL remel clt I [6] | 88.8 |
| G: T+ p1 -3 | 83.5 |
| H: T- [8] | |

### RUNCOV Performances

### -Analytical specificity

No positive responses were obtained when tested RUNCOV on the different nontarget viruses and particularly the other frequently circulating human Coronaviruses.

**[Table 3]**

| \/irus | RUNCOV Detection |
|---|---|
| Influenza A (H1N1) pdm 09 | 0/2 |
| Human Coronavirus -1 229E | 0/2 |
| Human Coronavirus -2 NL63 | 0/2 |
| Human Coronavirus -3 NL63 | 0/2 |
| Human Coronavirus -4 OC43 | 0/2 |
| Human Coronavirus -5 229E | 0/2 |
| Bat Alpha Coronavirus | 0/2 |
| Piq Coronavirus (DEP) | 0/2 |
| Poultrey Coronavirus (BIV) | 0/2 |

The RNAs extracted from the following viruses were tested in two replicates for this study. Apart the animal viruses, these are respiratory viruses causing clinical signs similar to those found during a SARS CoV-2 infection.

### -Analytical sensitivity (results obtained with a final RT-LAMP mix of 25 µL)

### Dilutions series of synthetic RNA in pure water or Amies swab

When tested on fiveten-fold dilutions (10 000 to 8 copies) of synthetic genomes of Sars-CoV-2 in pure water, RUNCOV displayed a very high sensitivity, with the lower concentrations (8 copies) being amplified for the 4 replicates (Tables 4 - dilution in RNAse-free pure water and Table 5 - Dilution in Amies negative swab³). For the same dilutions performed in negative swabs collected in Amies medium, the sensitivity threshold was raised to 40 copies, due to the inhibitory effects of the human matrix. The real-time qPCR realized on the same dilutions displayed Ct values of 39.6 for 8 genome copies and Ct values of 36.3 for 40 genomes copies.

**[Table 4]**

| | | **RT-LAMP RUNCOV** | | | | | | | | | |
|---|---|---|---|---|---|---|---|---|---|---|---|
| | | **Peak time (min)** | | | | **Annealing temperature (°C)** | | | | **Results** | |
| **Sample** | **copies nbr.** | **Rep.1** | **Rep.3** | **Rep. 3** | **Rep. 4** | **Rep.1** | **Rep.3** | **Rep. 3** | **Rep. 4** | | |
| RNA ctrl 2¹ | 1000 | 09:45 | 10:15 | 10:30 | NT | 83.6² | 83.5 | 83.7 | NT | **+** | **3/3** |
| RNA ctrl 2 | 200 | 12:00 | 12:45 | 12:15 | 13:45 | 83.7 | 83.7 | 83.7 | 83.6 | **+** | **4/**4 |
| RNA ctrl 2 | 40 | 15:15 | 15:45 | 18:15 | 16:15 | 83.7 | 83.5 | 83.7 | 83.7 | **+** | **4/4** |
| RNA ctrl 2 | 8 | 23:15 | 22:45 | 23:30 | 16:30 | 83.5 | 83.5 | 83.6 | 83.7 | **+** | **4/4** |

**[Table 5]**

| | | **RT-LAMP RUNCOV** | | | | | | | | | |
|---|---|---|---|---|---|---|---|---|---|---|---|
| | | **Peak time (min)** | | | | **Annealing temperature (°C)** | | | | **Results** | |
| **Sample** | **copies nbr.** | **Rep.1** | **Rep.3** | **Rep. 3** | **Rep. 4** | **Rep.1** | **Rep.3** | **Rep. 3** | **Rep. 4** | | |
| RNA ctrl 2 | 1000 | 13:00 | 13:45 | 14:00 | NT | 84.0 | 84.1 | 84.1 | NT | **+** | **3/3** |
| RNA ctrl 2 | 200 | 16:30 | 17:15 | 15:00 | 12:30 | 89.0 | 83.9 | 84.1 | 84.3 | **+** | **4/4** |
| RNA ctrl 2 | 40 | 17:30 | 23:30 | 17:00 | 19:30 | 84.2 | 84.1 | 89.1 | 83.9 | **+** | **4/4** |
| RNA ctrl 2 | 8 | - | - | - | - | - | - | - | - | - | **0/4** |

| | | | | | | | | | | | |
|---|---|---|---|---|---|---|---|---|---|---|---|
| NT: Not tested ¹ RNAshell control 2, 10 000 copies, ref GenBank: MN908947.3 ²only one annealing temperature corresponding to the highest peak was reported in this table. ³ the swab was previously treated with 1% 100X inactivation solution, heated at 95°C and cooled on ice before addition of RNA. | | | | | | | | | | | |

### Dilutions series of clinical samples in Amies or M4RT swabs

The limit of detection was also evaluated on three positive clinical samples serially diluted in negative Amies and/or M4RT swabs (Table 6). Threshold of LAMP detection for the different samples corresponded to Cts ranging between 33.6 and 35.9 when diluting in negative Amies swabs. A marked inhibitor effect on LAMP detection was shown for the same samples diluted in negative M4RT swabs. LAMP assay was at least 1000-fold less sensitive in this medium with a threshold of detection corresponding to Ct values ranging between 29.6 and 21.6. This negative effect of complex media has already been demonstrated.

### -Concentration/cleaning step and viral load measurement over time

In order to improve Sars-Cov-2 detection, a very simple and quick concentration step based on centrifugation was developed. Sars-Cov-2 is intra-cellular and the viral concentration increases when concentrating the human cells by centrifugation. This method is however very dependent of the state of the cells, which should be intact in order to keep the virus inside when pelleting the cells. It is recommended to use it when testing fresh samples or samples freeze in media that contains cryoprotectants.

This protocol associated with a cleaning step was particularly useful when detecting Sars-Cov-2 from the rich medium M4RT as described in Table 7. Viral load monitoring of a patient with a positive Covid-19 test showed that pelleting and wash steps suppressed inhibition compared to direct detection on crude sample, resulting in reliable signal recovery up to 12 days after symptom onset for samples with low viral load (Ct of 34.6). This method was also successfully applied to concentrate the virus in Amies swab samples (data not shown).

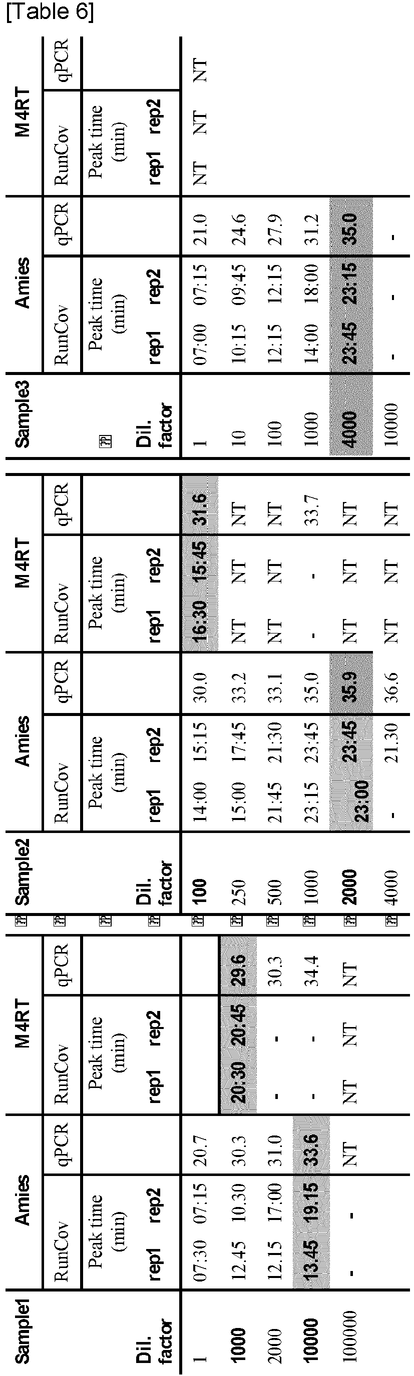

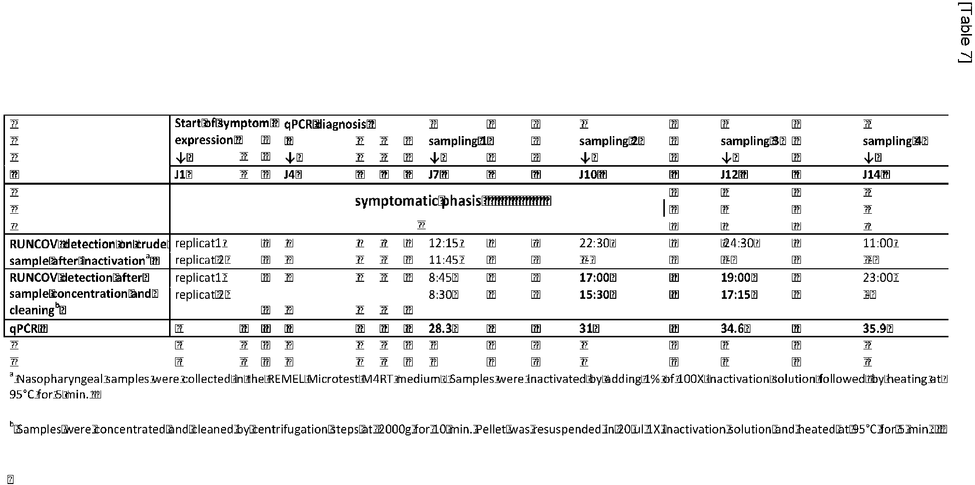

### -Diagnostic performance (results obtained with a final RT-LAMP mix of 25 µL)

Sensitivity and specificity values obtained for RUNCOV compared to RT-qPCR values on clinical samples were 88.1% [IC 95% 80,4-93,1] and 100 % [IC 95% 80.4-93.1], respectively.

**[Table 8]**

| Method | RT-qPCR+ | RT-qPCR- | total |
|---|---|---|---|
| RUNCOV + | 89 | 0 | 89 |
| RUNCOV- | 12 | 103 | 115 |
| Total | 101 | 103 | 204 |

The relation between Ct values and RUNCOV responses were analyzed in more detail (**Figure 4**). The 101 samples tested positive with RT-qPCR were classified according to their Ct (Dark curve). The corresponding RT-LAMP RUNCOV response was indicated by the vertical bars: dark for a positive response (2/2 replicates tested positive), light colored for an intermediate response (1/2 replicate tested positive) or a negative response (0/2 replicate tested positive). Except the two samples 74 and 76 with Ct values around 30 for which a positive RUNCOV response should have been obtained, the lack of a positive response starts for samples displaying Ct values around 33. Some samples with higher Ct values could also be amplified by RUNCOV. When considering the samples of epidemiological significance (Ct <35), the sensitivity of RUNCOV was raised to 95%.

For practical considerations all the samples were preserved at -80°C before their testing by RUNCOV. We think that better results could be obtained on fresh samples and a trial is currently setting up in collaboration with CHU, to test directly RUNCOV on fresh clinical samples, in parallel with RT-qPCR.

### Conclusion

The inventors developed a reliable and sensitive tool able to detect Sars-Cov-2 and its variants directly from the clinical samples, without the need of RNA extraction. It is an alternative molecular test for SARS-CoV-2 that can be easily deployed in the context of population screening, especially when access to standard RT-qPCR-based approaches is limited.

### Example 3- RUNCOV amplification: preliminary assays using saliva as a matrix

A sample consisting of Sars-CoV-2 culture filtrate was 100-fold diluted in water (control, displaying a Ct of 22.2 when tested by qPCR after RNA extraction) or in saliva. Different incubation time at 95°C (5 or 10 min) or different concentrations of the inactivation solution (1X or 10X) were tested in duplicate. The control was tested under standard RUNCOV process conditions. A positive result was obtained for each condition (i.e. acquisition of a peak time and at least one annealing temperature within the expected range). The best results in terms of fluorescent signal precocity were obtained using a 1X inactivation solution, as under standard conditions. Results are shown in the following table.

**[Table 9]**

| **Incubation time at 95°C (min)** | **Inact. sol. concentration** | **matrix** | **peak time** | **Ta (°C)** |
|---|---|---|---|---|
| 5 | 1 X | saliva | 12:00 | 89.3¹ |
| 5 | 1 X | saliva | 12:15 | 89.5 |
| 10 | 1 X | saliva | 12:00 | 89.5 |
| 10 | 1 X | saliva | 12:15 | 89.5 |
| 5 | 10X | saliva | 13:00 | 89.7 |
| 5 | 10X | saliva | 14:00 | 89.8 |
| 10 | 10X | saliva | 14:00 | 89.8 |
| 10 | 10X | saliva | 13:30 | 89.9 |
| 5 | 1 X | H20 | 12:45 | 89.9 |
| 5 | 1 X | H20 | 11:45 | 89.9 |

| | | | | |
|---|---|---|---|---|
| ¹only the highest annealing temperature peak values are reported here. | | | | |

These data confirm that the method according to the invention can be carried out from a saliva sample of a patient.

### Example 4- Detection of British variant using primers as set forth in SEQ ID NO: 1-6 and 8-13.

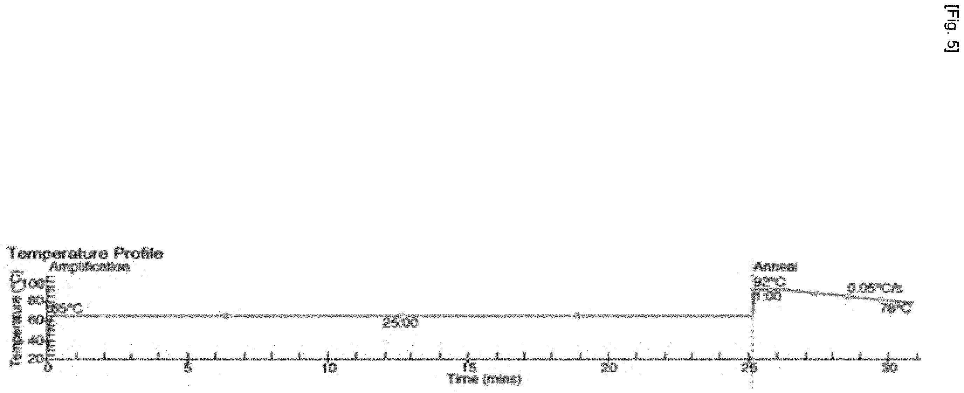

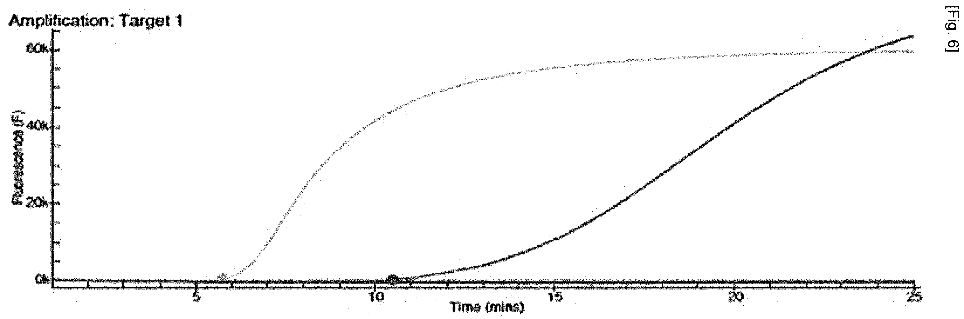

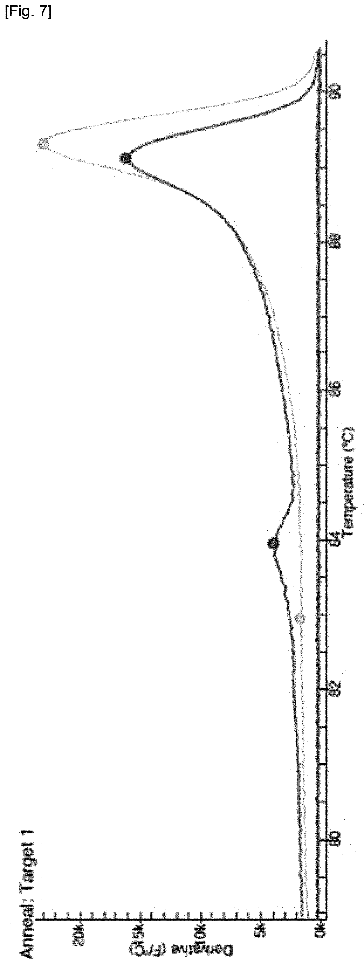

In this example, the inventors evaluated the efficacy of RUNCOV RT-LAMP amplification of a British variant clinical sample. The various samples were collected and processed during the trial carried out in collaboration with the CHU Reunion Sud (see above in the section "Tests on clinical samples" of Example 2). Results are shown in Figures 5-7, and summarized in the following table.

**[Table 10]**

| | sample | result | Time Peak value | Annealing temperature N | Annealing temperature S | qPCR result |
|---|---|---|---|---|---|---|
| 1 | 116 | negative | | | | <0 |
| 2 | 284 | negative | | | | <0 |
| 3 | 546 | negative | | | | <0 |
| 4 | 287 | **Positive** | 5:45 | 89.31°C | | >0 (Ct<23) |
| 5 | 673 | negative | | | | <0 |
| 6 | 450 | negative | | | | <0 |
| 7 | T + | **Positive** | 10:30 | 89.12°C | 83.96°C | |
| 8 | T - | negative | | | | |

Expression of results: time peak values (= time to results) are given for the two samples.

For the positive control, an annealing temperature is generated for both S and N regions unlike the British sample for which an annealing temperature is produced only for the N region. A sample will be considered positive if both a peak time value and at least one of the specific annealing temperatures (Ta) are obtained (83°C ≤Ta≤ 85.5°C for S and 87.5°C ≤Ta≤ 91°C for N). Thus, RUNCOV is able to detect a British variant even if it has a particular profile with regards to annealing temperature (only amplification of the N region).

### Example 5- Detection of South African variant using primers as set forth in SEQ ID NO: 1-6 and 8-13.

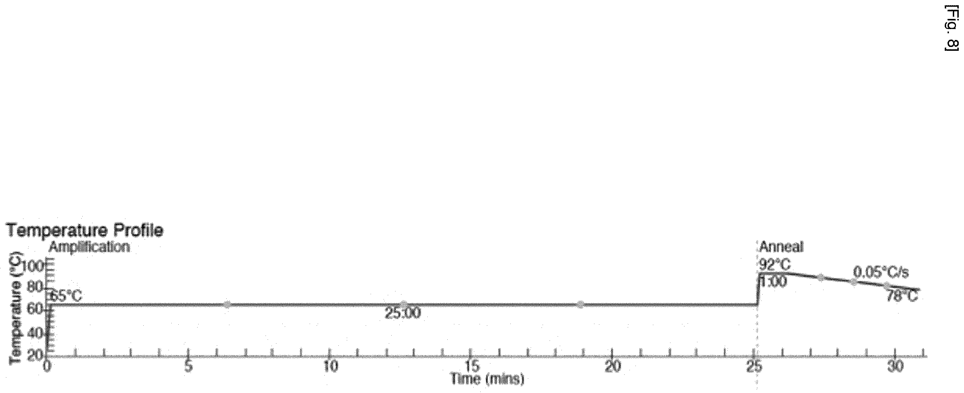

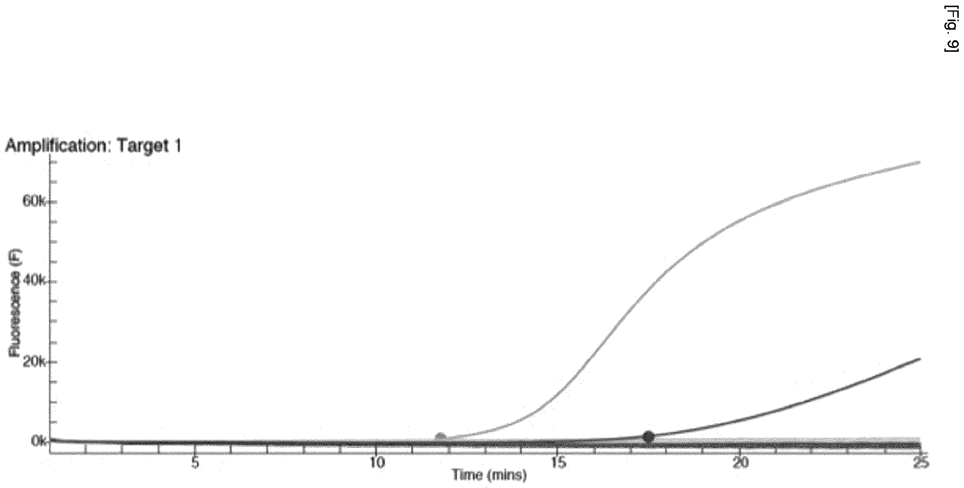

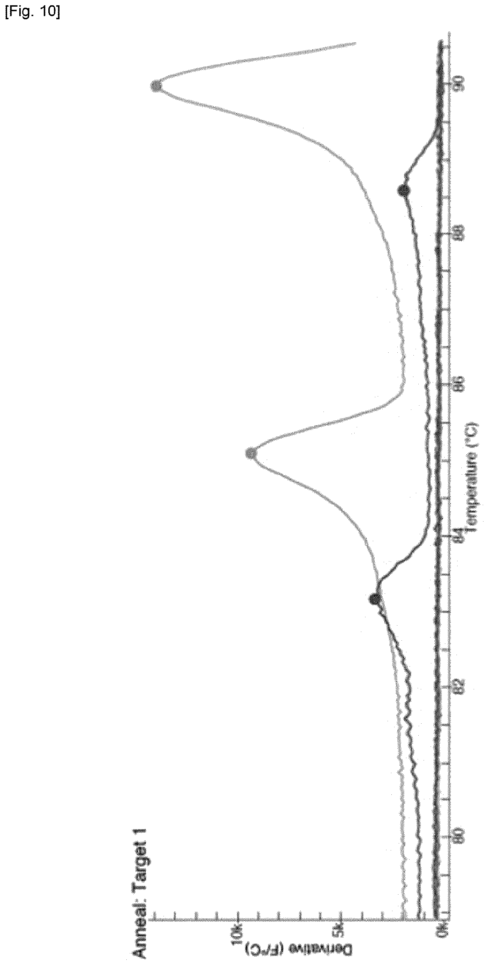

In this example, the inventors evaluated the efficacy of RUNCOV RT-LAMP amplification of a South African variant clinical sample. The various samples were collected and processed during the trial carried out in collaboration with the CHU Reunion Sud (see above in the section "Tests on clinical samples" of Example 2). Results are shown in Figures 8-10, and summarized in the following table.

| | sample | result | Time Peak value | Annealing temperature N | Annealing temperature S | qPCR result |
|---|---|---|---|---|---|---|
| 1 | 005 | negative | | | | <0 |
| 2 | 752 | **Positive** | 11:45 | 89.98°C | 85.10°C | >0 (Ct<33) |
| 3 | 990 | negative | | | | <0 |
| 4 | 219 | negative | | | | <0 |
| 5 | 547 | negative | | | | <0 |
| 6 | 627 | negative | | | | <0 |
| 7 | T + | **Positive** | 17:30 | 88.59°C | 83.17°C | |
| 8 | T - | negative | | | | |

For the two samples, an annealing temperature is generated for both S and N regions. A sample will be considered positive if both a peak time value and at least one of the specific annealing temperatures (Ta) are obtained (83°C ≤Ta≤ 85.5°C for S and 87.5°C ≤Ta≤91°C for N). RUNCOV is able to detect South African variants in the same way as for the original strain.

### Example 6- Detection of British variant using primers as set forth in SEQ ID NO: 1-7 and 8-13.

In this set of experiments, the inventors have evaluated the possibility to carry out the LAMP reaction by using both set of primers (S and N) but in which the S-primer set comprises 2 SPIR primers, one corresponding to the WT virus, one corresponding to the British variant.

Samples of UK variant (synthetic genome ref GenBank: EPI_ISL_710528 and of original strain (synthetic genome of Sars-Cov-2 ref GenBank: MN908947.3) were tested, at a concentration of 50 copies or 10 copies.

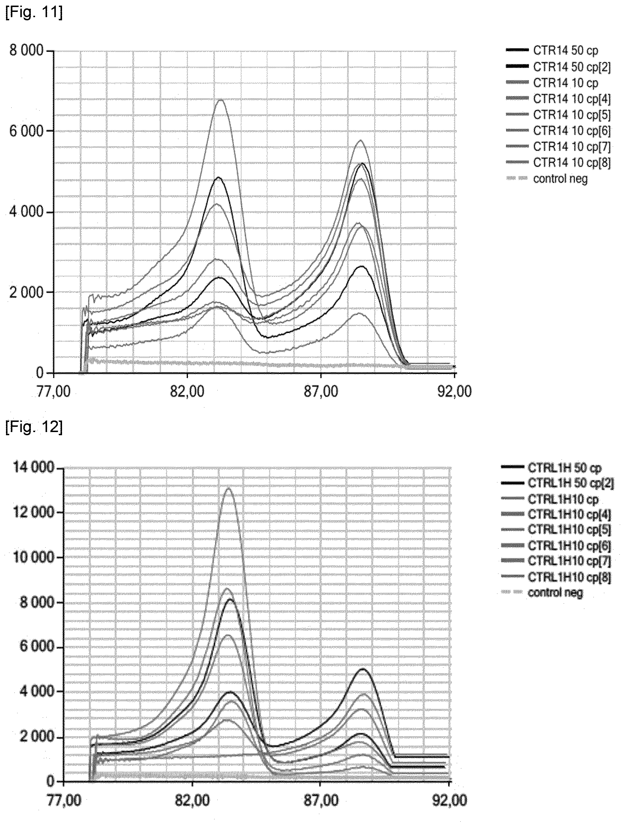

Results are shown in figures 11 (British variant) and 12 (original strain).

## Claims

1. An *in vitro* method of detecting the presence of a SARS coronavirus nucleic acid in a biological sample, the method comprising:
- contacting the biological sample with a sample buffer comprising a set of loop-mediated isothermal amplification primers specific for the SARS nucleic acid under conditions sufficient for a loop-mediated isothermal amplification, i.e. LAMP, of the SARS nucleic acid, wherein the set of LAMP primers comprises six primers:
- the six primers comprising or consisting of the nucleic acid sequences of SEQ ID NOs: 1-6; or
- the seven primers comprising or consisting of the variant nucleic acid sequences having at least 90% sequence identity to any one of SEQ ID NOs: 1-7, provided that variant nucleic acid sequences allow to carry out the LAMP;
thereby producing a SARS amplification sample;
- carrying out the LAMP from the SARS amplification sample, thereby producing a SARS amplification product; and
- detecting the SARS amplification product, thereby detecting the presence of the SARS nucleic acid in the sample.

2. The method according to claim 1, comprising prior to carrying out the LAMP from the SARS amplification sample,
- contacting the biological sample with a preparing sample, in order to obtain an inactivation mixture, and
- contacting the inactivation mixture with a sample buffer comprising a set of loop-mediated isothermal amplification primers specific for the SARS nucleic acid under conditions sufficient for a loop-mediated isothermal amplification, i.e. LAMP, of the SARS nucleic acid

3. The method according to claim 1 or 2, the set of LAMP primers comprising 7 primers as set forth in SEQ ID NO: 1-7 or SEQ ID NO: 1-6 and 14.

4. The method according to anyone of claim 1 or 2, wherein the set of LAMP primers comprises 13 primers comprising or consisting of the nucleic acid sequences of SEQ ID NOs: 1-13 or SEQ ID NO: 1-6 and SEQ ID NO: 8-14.

5. The method according to anyone of claim 1 or 3, wherein the sample buffer is lyophilised.

6. The method according to anyone of claims 2 to 4, wherein inactivation mixture is heated at 95°C, preferably for 5 min.

7. The method according to anyone of claims 1 to 5, further comprising contacting the SARS amplification sample with a reverse transcriptase under conditions sufficient for reverse transcription of the viral nucleic acid.

8. The method according to anyone of claims 1 to 6, wherein detecting the viral nucleic acid amplification product comprises the use of fluorescence detection.

9. The method according to any one of claims 1 to 7, wherein the sample comprises isolated DNA, isolated RNA, blood, plasma, serum, feces, saliva, nasopharyngeal sample.

10. A composition comprising at least one set of LAMP primers specific for a SARS nucleic acid, the set comprising seven primers comprising or consisting of the nucleic acid sequences of SEQ ID NOs: 1 to 6.

11. The composition of claim 10, further comprising one primer comprising or consisting of the nucleic acid sequences of SEQ ID NOs: 7 or SEQ ID NO: 14.

12. The composition of claim 10 or 11, further comprising six primers comprising or consisting of the nucleic acid sequences of SEQ ID NOs: 8 to 13.

13. A use of a composition according to anyone of claims 10 to 12, for diagnosing the presence of a SARS virus in a sample.

14. A kit comprising:
- a set of LAMP primers specific for a SARS nucleic acid comprising 7 primers comprising or consisting of the nucleic acid sequences of SEQ ID NOs: 1 to 7 or SEQ ID NO: 1-6 and 14; and
- a preparing buffer.

15. The kit according to claim 14, wherein the preparing buffer comprises tris(2-carboxyethyl)phosphine and ethylenediaminetetraacetic acid.
